# EUROPEAN PATENT APPLICATION

(11) **EP 2 103 588 A1**
(43) Date of publication of application: **23.09.2009**
(21) Application number: 08153084.2
(22) Date of filing: 20.03.2008
(51) Int. Cl.: C07C 17/26, C07C 25/18, C07D 235/18

(54) **2'-Halobiphenyl-4-yl intermediates in the synthesis of angiotensin II antagonists**

(71) Applicant: Lek Pharmaceuticals D.D., 1526 Ljubljana (SI)
(72) Inventor: The designation of the inventor has not yet been filed
(74) Representative: Kunic, Barbara

(57) **Abstract**

A process for obtaining 2'-halo-4-methylbiphenyls is described, which comprises reacting 4-halotoluene with a 1,2-dihalobenzene in the presence of elemental metal such as magnesium, lithium or zinc, wherein 0 to 0.9 molar, particularly 0 to 0.2 molar excess of 4-halotoluene in regard to 1,2-dihalobenzene is used, and arised organometal intermediates are quenched by elemental halogen. In addition, the coupling of arised 2'-halo-4-methylbiphenyls with 2-(1-propyl)-4-methyl-6-(1'-methylbenzimidazole-2-il)benzimidazole to afford 3'-(2'-halo-biphenyl-4-ylmethyl)-1,7'-dimethyl-2'-propyl-1H,3'H-[2,5']bibenzoimidazolyl, which can be further converted to organometallic compound and said organometallic compound is further reacted with formic acid derivative, such as N,N-dimethylformamide, alkylformiate or carbon dioxide to obtain telmisartan, is also described. Further described is use of in line analytics for monitoring the aforementioned reactions.

## Description

### Field of the Invention

The present invention relates in general to the field of organic chemistry and in particular to the preparation of substituted biphenyls, in particular to 4-halomethyl-2'-halobiphenyl and their use as intermediates in preparation of angiotensin II antagonists.

### Background of the Invention

Angiotensin II antagonists ("sartans") are efficient active compounds with biological activity which has proved useful for the treatment of hypertension. Most of commercially available sartans contain biphenyl moiety substituted with 5-tetrazolyl or carboxy group on the position 2' (Formula 1, X is COOH or 5-tetrazolyl).

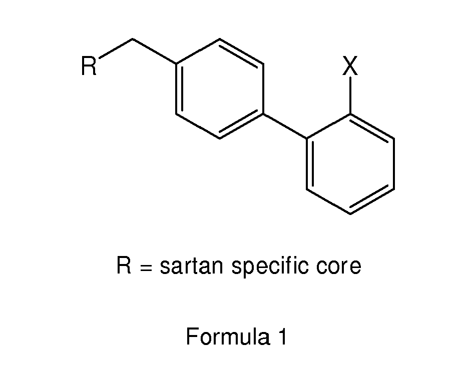

Telmisartan or salt or ester thereof (TLM, Formula 2) known from EP 502314 and which can be prepared in accordance with this invention is used as a pharmaceutical compound alone or in combination with pharmaceutically acceptable carrier for treatment of hypertension in human or animal and functions as angiotensin II antagonists.

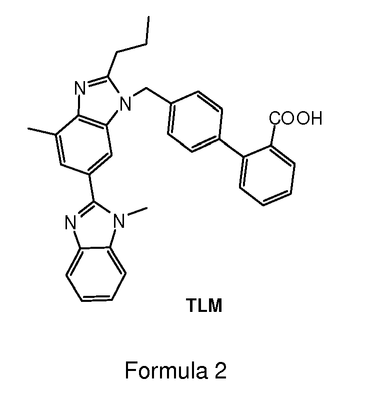

2'-halo-4-methylbiphenyls (Formula 3, X = Cl, Br, I) are potential starting materials for the synthesis of biphenyl type sartans but they have not found an application in industry due to inefficient or expensive synthesis. Known procedures use Suzuki and Heck couplings which need for industry unfriendly boron and tin compounds, Ullmann reaction (Chem. Rev. 2002, 102, 1359-1469) give low yields and coupling of Grignard intermediates require significant amounts of starting materials (Org. Lett. 2000, 2, 3675-3677). The later reaction was exercised using 1,2-dihalobenzene, 4-halotoluene in the presence of magnesium and iodine for quenching the Grignard intermediate wherein 1,2-dihalobenzene, 4-halotoluene and iodine were used in the ratio of 1:2:3, which is not economically favorable for industrial application.

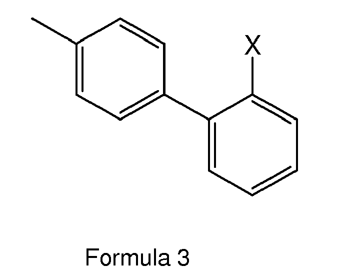

There is a need for efficient synthesis of biphenyl class of sartans especially for carboxy group containing derivatives such as telmisartan.

### Disclosure of the Invention

An aspect of the invention is a process for obtaining 2'-halo-4-methylbiphenyls, in which a 4-halotoluene is reacted with a 1,2-dihalobenzene in the presence of elemental metal such as magnesium, lithium or zinc, wherein 0 to 0.9 molar, particularly 0 to 0.2 molar excess of 4-halotoluene in regard to 1,2-dihalobenzene is used and arised organometal intermediates are quenched by elemental halogen. Proportionally for each 1 mol of 1,2-dihalobenzene used in the reaction less than 2.9 mol; particularly 1-2 mol; about 1 mol of elemental halogen is used to quench the organometal intermediate. The reaction is carried out in ether solvent or in a mixture of aprotic solvents comprising ether. Halo is selected from iodo, bromo or chloro.

In another aspect the invention is a process for obtaining 3'-(2'-halo-biphenyl-4-ylmethyl)-1,7'-dimethyl-2'-propyl-1H,3'H-[2,5']bibenzoimidazolyl (TLMH) characterized by coupling 2-(1-propyl)-4-methyl-6-(1'-methylbenzimidazole-2-yl)benzimidazole and 4'-halomethyl-2-halo-biphenyl.

In a further aspect the invention is a process for obtaining telmisartan **characterized in that** 3'-(2'-halo-biphenyl-4-ylmethyl)-1,7'-dimethyl-2'-propyl-1H,3'H-[2,5']bibenzoimidazolyl is converted to organometallic compound (TLMM); specifically where said conversion is preferably done with magnesium to Grignard reagent, or with Grignard reagent by halogen/metal-exchange reaction to another Grignard reagent, or with lithium to organolithium reagent, or with organolithium reagent by halogen/metal-exchange reaction to another organolithium or with zinc to organozinc reagent; and said organometallic compound is further reacted with formic acid derivative, such as N,N-dimethylformamide, alkylformiate or carbon dioxide; specifically where halo is selected from iodo, bromo or chloro, specifically iodo.

Another aspect of the invention is also use of compound 4'-halomethyl-2-halo-biphenyl for the synthesis of telmisartan, losartan, irbesartan, candesartan, olmesartan, valsartan, tasosartan or salt, ester or other derivative thereof.

Another aspect of the invention is use of formula TLMM for the synthesis of telmisartan or salt, ester or other derivative thereof.

In yet another aspect the invention is a compound of formula 4.

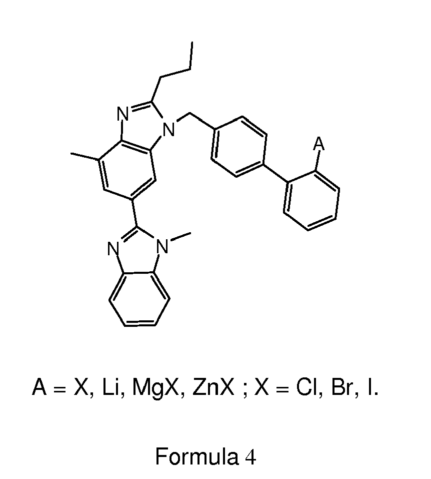

Another aspect of the invention is use of compound 4'-halomethyl-2-halo-biphenyl for the preparation of other sartans, such as losartan, irbesartan, candesartan, olmesartan, valsartan, tasosartan, salt, ester or other derivative thereof, or other compounds, that can be derived from the 4'-halomethyl-2-halo-biphenyl, wherein halo is selected from iodo, bromo or chloro and 4'-halomethyl-2-halo-biphenyl is not 4'-bromomethyl-2-iodo-biphenyl or 4'-bromomethyl-2-chloro-biphenyl.

Further aspect of the invention is in line analytic used to monitor the mentioned reactions and single components (reactants, intermediates, product and impurities) and/or for adjustment of reaction times or reaction temperatures in the said reactions.

### Detailed description of the Invention

Surprisingly it has been found that 2'-halo-4-methylbiphenyls can be obtained in a simple one-pot two-step technical process, in yields that are industrially applicable and competitive in which a 4-halotoluene is reacted with a 1,2-dihalobenzene in the presence of elemental metal, wherein 0 to 0.9 molar, particularly 0 to 0.2 molar excess of 4-halotoluene with regard to 1,2-dihalobenzene is used, that for each mol of 1,2-dihalobenzene, from 1.0 to 1.9, particularly from 1.0 to 1.2 mol 4-halotoluene is used, more particularly from 1.05 to 1.15 mol, and arised organometal intermediates are quenched proportionally for each 1 mol of 1,2-dihalobenzene by less than 2.9 mol, particularly 1-2 mol, about 1 mol of elemental halogen.

The present invention provides a process for obtaining 2'-halo-4-methylbiphenyl in which 1 to 4 eq. of 4-halotoluene is dissolved in an aprotic solvent, which may be selected from tetrahydrofuran, methyltetrahydrofuran, diethylether, diisopropylether, methyl tertiary butyl ether, dibutyl ether or diphenyl ether and the solution is maintained at about 15 °C to 80 °C, preferably at room temperature. 4-halotoluene can be selected from p-bromotoluene, p-chlorotoluene or p-iodotoluene. 2 to 5 eq of metal is added and stirred for about 5 to 180 minutes. By the term metal there is contemplated any embodiment capable of forming organometal intermediates such as, e. g. magnesium, lithium or zinc. To thus prepared mixture one adds 1 eq. of 1,2-dihalobenzene dissolved in up to in the said solvent dropwise at 15 °C to 80 °C, preferably at 50 to 60 °C during 1 min to 5 hours, such as during 2 hours and stirred at same temperature for 1 to 48 hours. Dihalobenzene can be selected from 1-bromo-2-chlorobenzene, 1-chloro-2-iodobenzene, 1-bromo-2-iodobenzene, 1,2-dibromobenzene, or 1,2-diiodobenzene. To the solution of thus obtained 4'-methyl-biphenyl-2-ylmetal halide in the said solvent 1 to 5 eq of elemental halogen is added at 15 °C to 80 °C, preferably at room temperature. 4'-methyl-biphenyl-2-ylmetal halide can be any appropriate organometal compound such as, e. g. 4'-methyl-biphenyl-2-ylmagnesium halide, wherein halide may be iodide, bromide or chloride, preferably bromide. Elemental halogen is selected from iodine (I₂) or bromine (Br₂) or chlorine (Cl₂). The mixture is stirred for minimum 5 minutes. The remaining halogen is reduced with aqueous solution of NaHSO₃ or Na₂S₂O₃. After work-up with water and an apolar solvent which may be selected from esters, ethers, chlorinated solvents and hydrocarbons preferably from aliphatic hydrocarbons such as n-hexane, n-pentane, n-heptane, cyclohexane, methylcyclohexane are added. The phases are separated and the organic phase is evaporated. The product is for example purified with LPLC chromatography. Mobile phase is n-hexane, stationary phase is silica gel 60. The fractions are followed by TLC method and collected. The main fractions are evaporated to give 2-halo-4'methyl-biphenyl in yield off at least 60 %.

In a more specific but preferred example 1.1 eq of 4-bromotoluene is dissolved in 5 to 7 times bigger volume of tetrahydrofuran and the solution is maintained at room temperature. 2.5 eq of Mg is added and stirred for at least 30 minutes. To thus prepared mixture one adds 1 eq of 1-bromo-2-chlorobenzene diluted by the same volume of tetrahydrofurane dropwise at 55 °C during 2 hours and stirred at same temperature for 1 to 3 hours. To the solution of thus obtained 4'-methyl-biphenyl-2-ylmagnesium bromide in tetrahydrofuran 1 eq of iodine (I₂) is added and the reaction is worked up as described above.

The employment of excess of only 0.1 eq of 4-halotoluene dramatically improves the yield, which exceeds in this specific example 60 %. Furthermore, only 1 eq of halogen was sufficient to quench the reaction, which lasts 3 hours at most. The procedure is a great improvement of known literature procedures (Org. Lett. 2000, 2, 3675-3677) which takes 14 hours to react and uses over two times excess of 4-halotoluene and three times excess of halogen to achieve comparable yields.

In additional reactions obtained 2-halo-4'methyl-biphenyl is halogenated in dichloromethane yielding 4'-halomethyl-2-halo-biphenyl. Specific compounds thus prepared are 4'-bromomethyl-2-bromo-biphenyl, 4'-iodomethyl-2-iodo-biphenyl, 4'-iodomethyl-2-bromobiphenyl, 4'-iodomethyl-2-chloro-biphenyl, 4'-chloromethyl-2-iodo-biphenyl, 4'-chloromethyl-2-bromo-biphenyl, 4'-chloromethyl-2-chloro-biphenyl.

In another embodiment the invention also provides for coupling of 2-halo-4'methyl-biphenyl and 2-(1-propyl)-4-methyl-6-(1'-methylbenzimidazole-2-il)benzimidazole, which is performed but not limited as follows: 10 - 25 mL of sulfolane (tetramethylene sulfone) or *N,N-*dimethylacetamide or *N*-methyl-2-pyrrolidone or dimethyl sulfoxide is charged to the flask. 0.85 g of 2-(1-propyl)-4-methyl-6-(1'-methylbenzimidazole-2-il)benzimidazole and 0.38 g of strong base such as K*t*BuO or appropriate amount of NaOH, KOH, LiOH, Na₂CO₃, K₂CO₃, NaHCO₃ or KHCO₃ are added. The mixture is brought to the temperature of about 25 °C - 45 °C to dissolve all the components. The solution is then brought to the temperature of about 5 °C to 25 °C and 1.07 g of previously obtained product in 5 to 20 mL of solvent is added during 0.5 - 5 hours. The reaction mixture is stirred at the same temperature for additional 0 - 5 hours. 40 mL of demineralised water and 35 mL of ethyl acetate are added. The phases are separated. The EtOAc phase is washed several times with saturated water solution of NaCl. The solvent is evaporated and 4 mL mixture of EtOAc and acetone is added. The suspension is stirred for 15 minutes to 5 hours. The precipitate is filtered off and dried to give TLMH.

In a preferred example telmisartan is prepared from the intermediate 3'-(2'-halo-biphenyl-4-ylmethyl)-1,7'-dimethyl-2'-propyl-1 H,3'H-[2,5']bibenzoimidazolyl (TLMH) by introducing C-1 synthon in high oxidation state in one-pot two-step procedure on the position 2'. Scheme 1 represents the synthesis of TLMH.

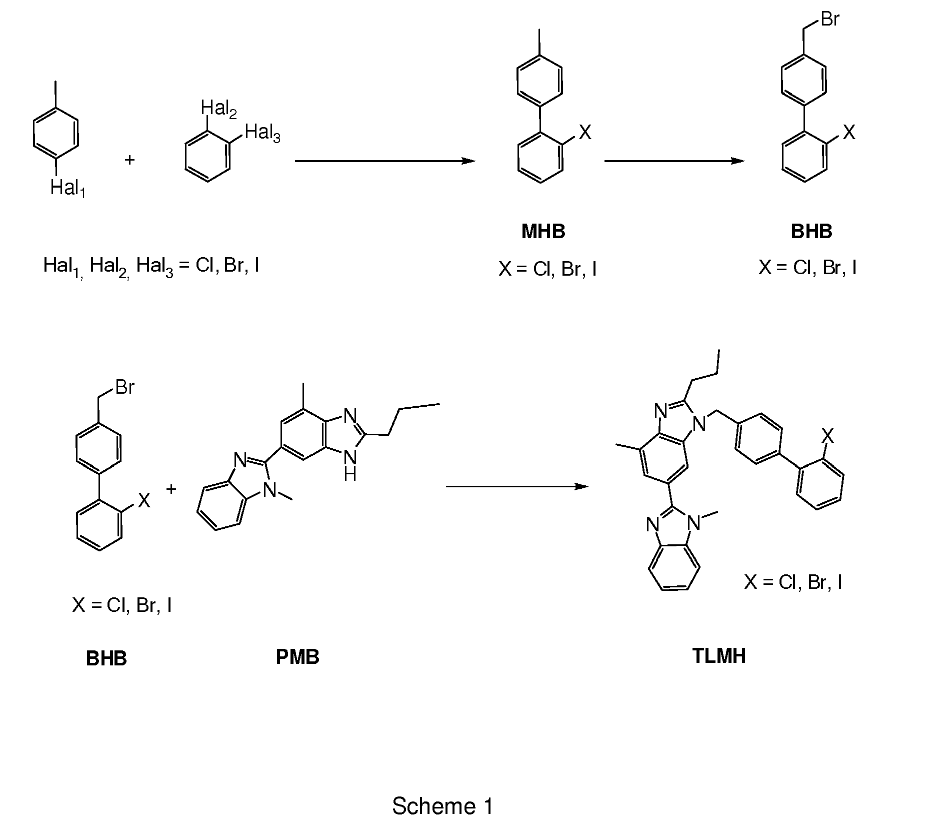

TLMH is further converted to organometallic compound TLMM as presented in Scheme 2 which is further reacted with formic acid derivative such as esters and amides, preferably N,N-dimethylformamide, or carbon dioxide in the second step as presented in Scheme 3.

Formula 4 encompasses embodiments TLMH and TLMM, wherein TLMH is halo substituted on the 2' position of 3'-(biphenyl-4-ylmethyl)-1,7'-dimethyl-2'-propyl-1H,3'H-[2,5']bibenzoimidazolyl, representing 3'-(2'-halo-biphenyl-4-ylmethyl)-1,7'-dimethyl-2'-propyl-1H,3'H-[2,5'] bibenzoimidazolyl, and TLMM is Li, MgX or ZnX substituted on the 2' position of the biphenyl part of 3'-(biphenyl-4-ylmethyl)-1,7'-dimethyl-2'-propyl-1H,3'H-[2,5']bibenzoimidazolyl, wherein X is selected from iodo, bromo or chloro. Metals for creating organometallic intermediate are selected from alkali, earth-alkali, transition metals or lantanides. In created organometallic compounds of type TLM-Met, TLM-MetX or (TLM)₂-Met TLM means decarboxytelmisartan radical, Met is metal (monovalent or bivalent respectively) and X is chloro, bromo or iodo. Preferred metals are lithium, magnesium or zinc.

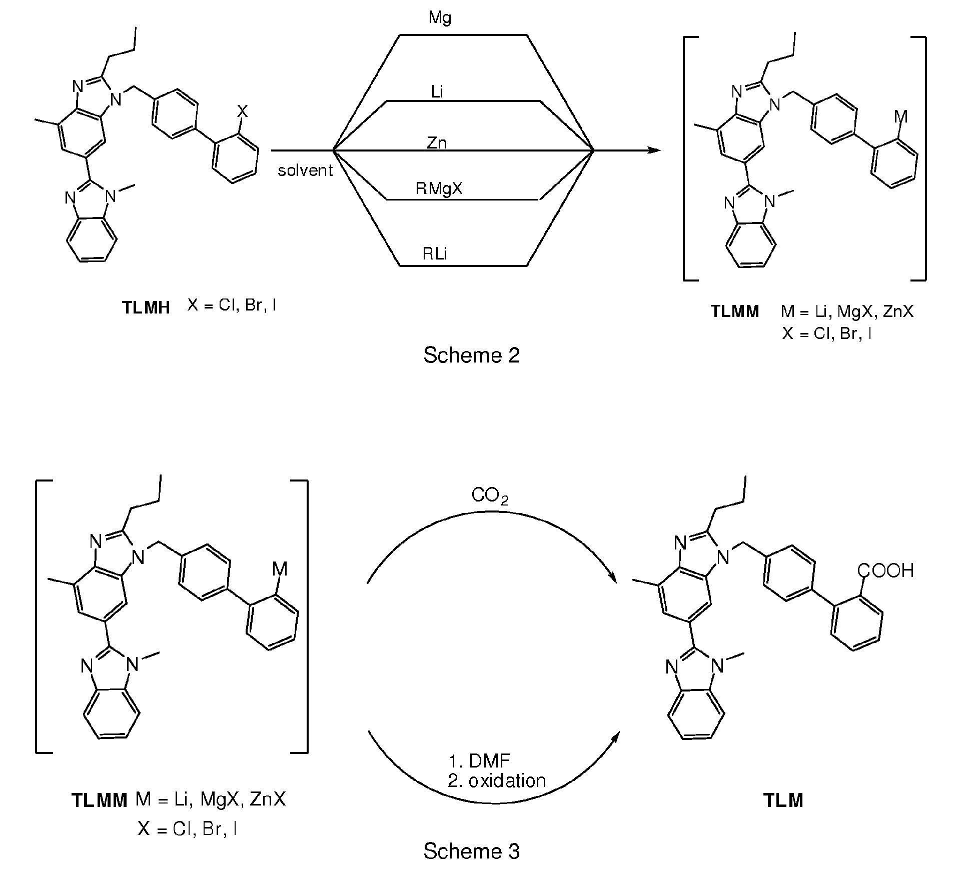

Organomagnesium compounds are preferably prepared as Grignard reagents of type TLM-MgX (X = Cl, Br, I) by reacting magnesium with the halo compound TLMH optionally in the presence of a catalyst preferably iodine or 1,2-dibromoethane. Alternatively Grignard intermediates TLM-MgX are prepared with halogen/metal-exchange reaction with another commercial Grignard reagent.

Organolithium reagent of type TLM-Li is preferably prepared from TLMH by halogen/metal-exchange reaction with elemental lithium or by translithiation with another organolithium compound preferably with alkyllithium most preferably butyllithium.

Organozinc reagents of type TLM-ZnX (X is selected from iodo, bromo or chloro) can be prepared from TLMH and zinc or its derivative but are preferably prepared from organometal intermediates of type TLM-MgX or TLM-Li and anhydrous zinc halogenide selected from chloride, bromide or iodide.

Organometallic compound is preferably prepared in situ in anhydrous solvents preferably selected from ethers and is converted to telmisartan by bubbling of carbon dioxide or stirred with a solid carbon dioxide (dry ice) at temperature from -50 ºC to boiling point, preferably at room temperature for 5 min to 10 h preferably 2 to 4 hours.

In one workup Grignard reagent is formed in tetrahydrofuran or methyltetrahydrofuran or diethylether or diisopropylether or methyl tertiary butyl ether or diphenyl ether by charging magnesium turnings and catalytic amount of iodine or 1,2-dibromoethane and adding and dissolving TLMH in an above mentioned ether solvent. The reaction mixture is stirred for 0.5 - 10 hours. Following that the prepared solution is bubbled with carbon dioxide gas (CO₂) or stirred with a solid carbon dioxide (dry ice) for 5 to 300 minutes. The diluted HCl is added and telmisartan is precipitated and filtered off. The cake is washed with water and telmisartan isolated.

In another special but not limited example a solution of TLMH in an above mentioned ether solvent is treated with alkyl lithium preferably butyl lithium at the temperature bellow 0 ºC, preferably bellow -40 ºC to provide halogen litium exchange. Following that the prepared solution is bubbled with carbon dioxide gas (CO₂) or stirred with a solid carbon dioxide (dry ice) for 5 to 300 minutes. The diluted HCI is added and telmisartan is precipitated and filtered off. The cake is washed with water and telmisartan isolated.

In the alternative reaction the above described organometallic intermediates are treated by formic acid derivatives such as esters and amides, preferably N,N-dimethylformamide to give telmisartan aldehyde precursor which can be further oxidized to telmisartan.

In a special but not limited example the above described organolithium solution is quenched with N,N-dimethylformamide at the temperature bellow 0 ºC, preferably slowly rising temperature from bellow -40 ºC to room temperature to obtain 3'-(2'-formyl-biphenyl-4-ylmethyl)-1,7'-dimethyl-2'-propyl-1H,3'H-[2,5']bibenzoimidazolyl which is converted to telmisartan by oxidation with manganese like manganese (IV) oxide and potassium permanganate, chromium (VI) oxidants like sodium chromate or chromium (VI) oxide adducts, peroxides like hydrogene peroxide in water, low alkanoic acids or alcohols or organic peroxides like alkyl peroxides or carboxylic peroxoacids or higher oxidation state chlorine compounds like metal or organic hypochlorites or sodium chlorate (III) or bromo compounds like bromine, N-bromosuccinimide, inorganic peroxo salts like potassium peroxodisulfate, potassium peroxomonosulfate known under tradename OXONE® or sodium perborate or silver oxyde or oxygene or the like. Preferably sodium chlorate (III) with hydrogene peroxide is used.

In another embodiment the invention also provides for simple preparation of other angiotensin II antagonists like losartan, irbesartan, candesartan, olmesartan and valsartan, which have a residue such as heterocycle or amino acid derivative via methylene linked to a biphenyl and which include a tetrazole substituent on biphenyl in - lieu of carboxylic acid. For those 4'-halomethyl-2-halo-biphenyl is coupled with appropriate residue, which could be prepared or obtained as defined by general knowledge and well-known to the person versed in the art, and biphenylic halo substituent converted into tetrazole as shown in Scheme 4.

Base can be selected from inorganic base such as, sodium hydroxide, potassium hydroxide and the like. R₃ in the Scheme 4 represents alkyl.

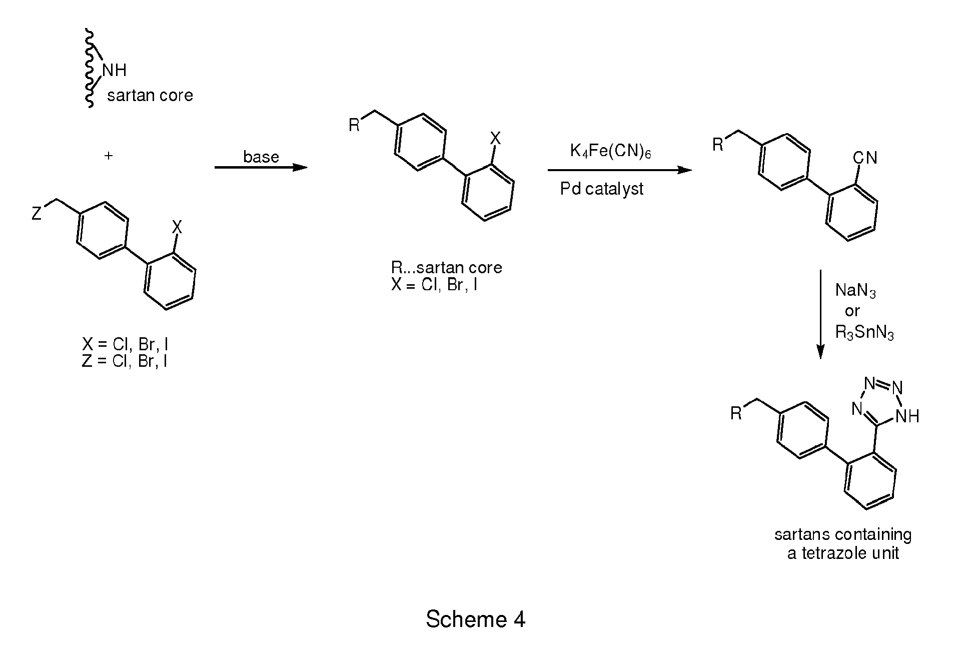

The overall processes of present invention utilize series of Grignard reactions which allows unification of solvents and solvent conditions, furthermore the concentration of components in the reaction mixture is in an aspect of the invention advantageously monitored by the use of in line FTIR analytic, thus allowing the adjustment of reaction times and temperatures.

In line analytic (ATR FTIR probe) is used to monitor the reactions and single components (reactants, intermediates, product and impurities) and/or to adjust reaction time or reaction temperatures. The background of air is used. The following peaks (heights calculated to single baseline) are monitored: 3242cm⁻¹, 2339 cm⁻¹, 1509 cm⁻¹, 1486 cm⁻¹, 1405 cm⁻¹, 1393 cm⁻¹, 1227 cm⁻¹, 1039 cm⁻¹, 1034 cm⁻¹, 883 cm⁻¹, 880 cm⁻¹, 826 cm⁻¹, 802 cm⁻¹, 783 cm⁻¹, 752 cm⁻¹, 764 cm⁻¹, 721 cm⁻¹, 694 cm⁻¹. The 1^{st}and 2^{nd} derivative tools and solvent subtraction tools are used to isolate the component peaks from the IR spectrum of the reaction mixture.

The peaks at 783 cm⁻¹ and 1227 cm⁻¹ represent the p-toluyl magnesium halide, the peaks at 1035 cm⁻¹ and 880 cm⁻¹ represent the ether complex of a Grignard and the peak at 695 cm⁻¹ is intermediate (1-halophenyl-2-magnesium halide). The reaction is finished when the peak at 695 cm⁻¹ disappears.

Additionally or alternatively the redox electrode is used to trace the concentration of iodine, bromine or chlorine.

The following examples illustrate the present invention and are not intended to limit the scope of the invention.

### Example 1:

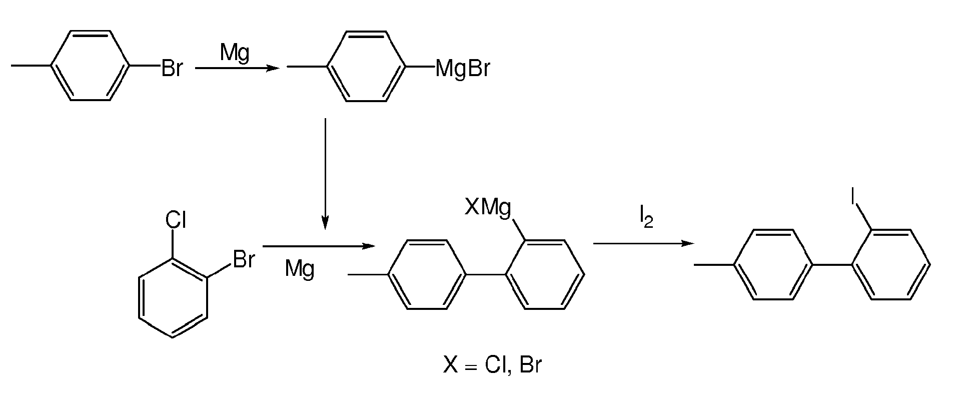

### Formation of Grignard reagent

60 mL of tetrahydrofuran is charged to the flask. 9 g (52.6 mmol) of p-bromotoluene is added and the solution is maintained at 20°C. 3.0 g (125 mmol) of Mg is added and stirred for minimal 30 minutes.

### Coupling

9.3 g (48.7 mmol) of 1-bromo-2-chlorobenzene in 5 mL of THF is added into the prepared reaction mixture of p-toluyl magnesium bromide and remaining magnesium at 55 °C in 2 hours and stirred at 55 °C for 2 hours.

### Quenching

The prepared solution of 2-magnesium bromide-4'-methyl-biphenyl is cooled to room temperature, and 20 mL of tetrahydrofuran with further 12.3 g (48,7 mmol) of iodine (I₂) are added. The mixture is agitated for minimum 5 minutes. The remaining iodine in neutralised with aqueous solution of NaHSO₃. 70 mL of demineralised water and 50 mL of n-hexane are added. The phases are separated and the upper (n-hexane) phase is evaporated. The 15.6 g of yellowish liquid is obtained.

The product is purified with LPLC chromatography. Mobile phase is n-hexane, stationary phase is silicagel 60. The fractions are collected. The main fractions are evaporated. 8.6 g (60 %) of 2-iodo-4'methyl-biphenyl (colourless liquid) is obtained.

¹H NMR (300 MHz, CDCl₃) δ: 2.56 (s, 3H), 7.13 (dt, *J* = 7.5 Hz, *J* = 2.0 Hz, 1 H), 7.39 (s, 4H), 7.44 (dd, *J* = 7.6 Hz, *J* = 1.9 Hz, 1 H), 7.47 - 7.52 (m, 1 H), 8.09 (dd, *J* = 7.9 Hz, *J* = 1.0 Hz, 1 H). ¹³C NMR (75 MHz, CDCl₃) δ: 146.4, 141.2, 139.3, 137.1, 130.0, 129.0, 128.5, 128.5, 128.0, 98.8, 21.2.

### Example 2: Bromination of 2-iodo-4'methyl-biphenyl

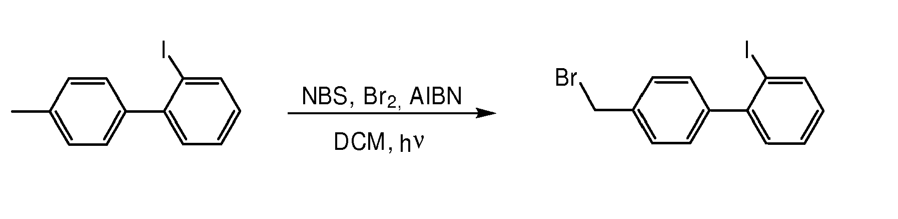

20 mL of dichloromethane (DCM) is charged to the flask. 0.73 g of N-bromosuccinimide (NBS), 0.08 g of 2,2'-azoisobutyronitrile (AIBN), 18 µL of Br₂ and 0.9 g of 2-iodo-4'-methylbiphenyl are added. The reaction is carried out under reflux temperature for minimum 2 hours and the flask is lighted all the time. The reaction is quenched with aqueous solution of Na₂S₂O₃. The phases are separated and the lower DCM phase is washed with demineralised water one more time. The DCM phase is evaporated and 4 mL of n-hexane is charged and stirred at room temperature for 30 minutes. The suspension is then cooled to 0 °C and filtered. The cake is washed with 2 mL of solvent. 0.6 g of white crystals of 4'-bromomethyl-2-iodo-biphenyl is obtained.

¹H NMR (300 MHz, CDCl₃) δ: 4.58 (s, 2H), 7.06 (dt, *J* = 7.6 Hz, *J* = 1.8 Hz, 1 H), 7.31 (dd, *J* = 7.7 Hz, *J* = 1.8 Hz, 1H), 7.35 (d, *J* = 8.2 Hz, 2H), 7.40 (dt, *J* = 7.4 Hz, *J* = 1.2 Hz, 1H), 7.48 (d, *J* = 8.2 Hz, 2H), 7.98 (dd, *J* = 7.9 Hz, *J* = 1.1 Hz, 1H). ¹³C NMR (75 MHz, CDCl₃) δ: 145.8, 144.1, 139.5, 137.0, 130.0, 129.6, 128.9, 128.6, 128.1, 98.3, 33.2.

### Example 3: Alkylation of (2-(1-propyl)-4-methyl-6-(1'-methylbenzimidazole-2-il)benzimidazole)

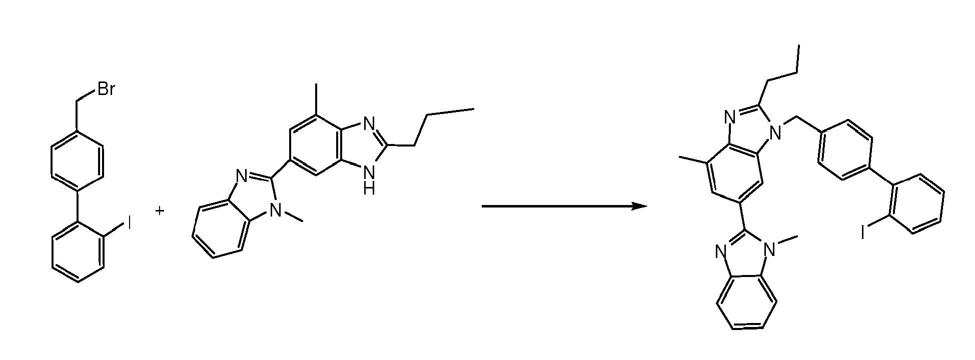

15 mL of sulfolane (tetramethylene sulfone) is charged to the flask. 0.85 g of PMB (2-(1-propyl)-4-methyl-6-(1'-methylbenzimidazole-2-il)benzimidazole) and 0.38 g of potassium tert-butoxide are added. The mixture is heated above 30 °C to dissolve all the components. The solution is than cooled down to 15 °C and 1.07 g of 4'-bromomethyl-2-iodo-biphenyl in 5 mL of solvent is added slowly during 45 minutes. The reaction mixture is stirred at the same temperature for additional 2 hours. 40 mL of demineralised water and 35 mL of EtOAc (ethyl acetate) are added. The phases are separated. The upper EtOAc phase is washed several times with saturated water solution of NaCl. The solvent is evaporated and 4 mL mixture of EtOAc and acetone is added. The suspension is stirred for 30 minutes at room temperature. The suspension is filtered and 0.94 g of white crystals of 3'-(2'-iodo-biphenyl-4-ylmethyl)-1,7'-dimethyl-2'-propyl-1 H,3'H-[2,5']bibenzimidazolyl are obtained.

¹H NMR (300 MHz, CDCl₃) δ: 1.06 (t, *J* = 7.4 Hz, 3H), 1.84 - 1.92 (m, 2H), 2.95 (t, *J* = 7.8 Hz, 2H), 3.81 (s, 3H), 5.46 (s, 2H), 7.03 (ddd, *J* = 7.9 Hz, *J* = 7.4 Hz, *J* = 1.8 Hz, 1H), 7.13 (d, *J* = 8.0 Hz, 2H), 7.23 - 7.54 (m, 9H), 7.77 - 7.83 (m, 1H), 7.94 (dd, *J* = 7.9 Hz, *J* = 1.1 Hz, 1H).

### Example 4: Formation of Grignard reagent of 3'-(2'-iodo-biphenyl-4-ylmethyl)-1 ,7'-dimethyl-2'-propyl-1 H,3'H-[2,5']bibenzoimidazolyl and synthesis of telmisartan

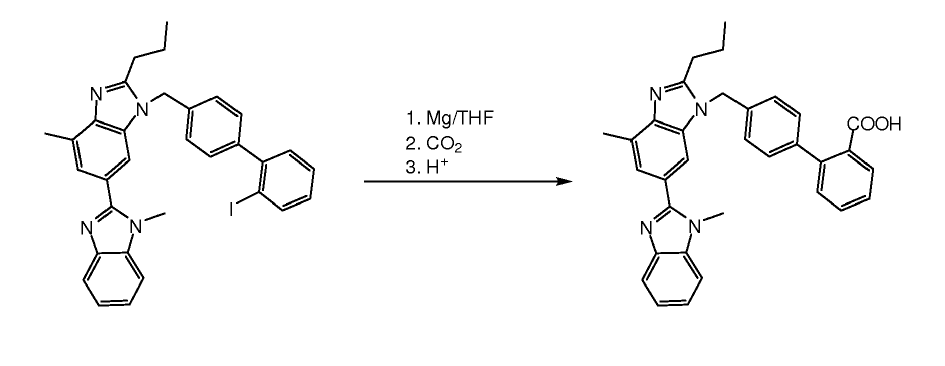

20 mL of THF is charged into the flask. 0.05 g of Mg and catalytic amount of iodine or 1,2-dibromoethane is added. 0.5 g of 3'-(2'-iodo-biphenyl-4-ylmethyl)-1 ,7'-dimethyl-2'-propyl-1H,3'H-[2,5']bibenzoimidazolyl in 10 mL of solvent is added and reaction mixture is stirred for 3 hours at room temperature. The solution of (4'-((1,7'-dimethyl-2'-propyl-1H,3'H-2,5'-bibenzo[d]imidazol-3'-yl)methyl)biphenyl-2-yl)magnesium iodide is obtained.

The prepared solution of (4'-((1,7'-dimethyl-2'-propyl-1H,3'H-2,5'-bibenzo[d]imidazol-3'-yl)methyl)biphenyl-2-yl)magnesium iodide is bubbled with carbon dioxide (CO₂) for 3 hours at room temperature. The diluted HCI is added and precipitated telmisartan is filtered of. The cake is washed with water. The 0.4 g of crude telmisartan (2-[4-[[4-methyl-6-(1-methylbenzoimidazol-2-yl)-2-propyl-benzoimidazol-1-yl]methyl]phenyl]benzoic acid) is obtained.

### Example 5: Formation of 3'-(2'-formyl-biphenyl-4-ylmethyl)-1,7'-dimethyl-2'-propyl-1H,3'H-[2,5']bibenzoimidazolyl and synthesis of telmisartan

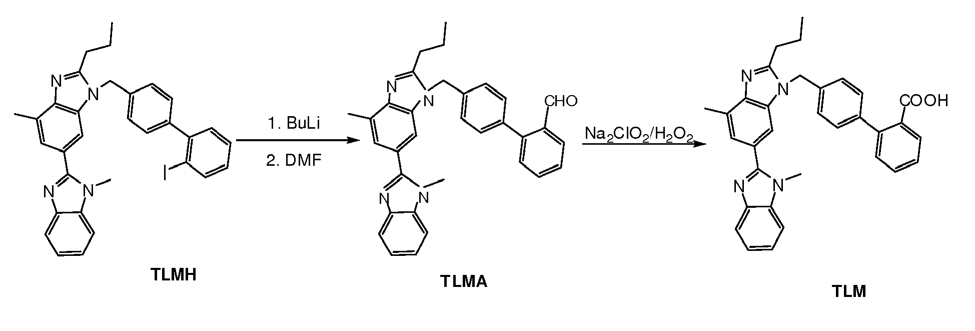

To a suspension of 3'-(2'-iodo-biphenyl-4-ylmethyl)-1,7'-dimethyl-2'-propyl-1H,3'H-[2,5']bibenzoimidazolyl (460 mg) in toluene (13 mL) is added 1.6 M BuLi in hexane (0.56 mL) within 60 min at room temperature. After 60 h, dimethylformamide (DMF; 0.2 mL) is added all at once and the mixture is stirred for an additional 24 h. Water (4 mL) is added, the phases are separated and extracted 4 times with 9 mL of ethyl acetate. Combined organic extracts are dried over MgSO₄ and evaporated under reduced pressure to obtain 230 mg of TLMA as crude amorphous product.

A solution of 156 mg of NaClO₂X4H₂O in 1 mL of water was added dropwise by canilla to a stirred mixture of 4'-[(2-n-propyl-4-methyl-6-(1-methyl-imidazol-4-yl)-benzimidazol-1-yl)methyl]-biphenyl-2-carboxaldehyde (230 mg) in 1 mL of acetonitrile, 0.16 ml of 10 % solution of NaH₂PO₄ in water and 0.23 mL of 30 % H₂O₂ at the temperature 10 °C. pH of the mixture was adjusted to 2 with concentrated hydrochloric acid. Stirring was continued for 1.5 h at room temperature. Reaction mixture was poured in 1.2 mL of water, stirred for 15 minutes, product filtered, washed with water and dried *in vacuo* to yield 210 mg of telmisartan.

## Claims

1. A process for obtaining 2'-halo-4-methylbiphenyls, **characterized in that** a 4-halotoluene is reacted with a 1,2-dihalobenzene in the presence of elemental metal such as magnesium, lithium or zinc, wherein 0 to 0.9 molar, particularly 0 to 0.2 molar excess of 4-halotoluene in regard to 1,2-dihalobenzene is used, and arised organometal intermediates are quenched by elemental halogen.

2. The process according to Claim 1, wherein proportionally for each 1 mol of 1,2-dihalobenzene used less than 2.9 mol; particularly 1-2 mol; about 1 mol of elemental halogen is used to quench the organometal intermediate.

3. The process according to Claim 1 or 2, **characterized in that** the reaction is carried out in ether solvent or in a mixture of aprotic solvents comprising ether.

4. A process for obtaining 3'-(2'-halo-biphenyl-4-ylmethyl)-1,7'-dimethyl-2'-propyl-1H,3'H-[2,5']bibenzoimidazolyl **characterized by** coupling 2-(1-propyl)-4-methyl-6-(1'-methylbenzimidazole-2-il)benzimidazole and 4'-halomethyl-2-halo-biphenyl.

5. A process for obtaining telmisartan or salt thereof, **characterized in that** 3'-(2'-halo-biphenyl-4-ylmethyl)-1,7'-dimethyl-2'-propyl-1 H,3'H-[2,5']bibenzoimidazolyl is converted to organometallic compound and said organometallic compound is further reacted with formic acid derivative, such as N,N-dimethylformamide, alkylformiate or carbon dioxide.

6. The process according to Claim 5, **characterized in that** 3'-(2'-halo-biphenyl-4-ylmethyl)-1,7'-dimethyl-2'-propyl-1H,3'H-[2,5']bibenzoimidazolyl is converted with magnesium to Grignard reagent, or with Grignard reagent by halogen/metal-exchange reaction to another Grignard reagent, or with lithium to organolithium reagent, or with organolithium reagent by halogen/metal-exchange reaction to another organolithium or with zinc to organozinc reagent.

7. A compound of formula 4: 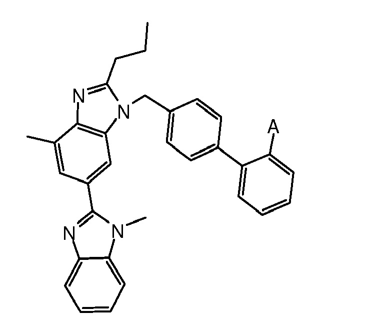 wherein A represents X, Li, MgX or ZnX and X represents Cl, Br or I.

8. A compound selected from 4'-bromomethyl-2-bromo-biphenyl, 4'-iodomethyl-2-iodo-biphenyl, 4'-iodomethyl-2-bromo-biphenyl, 4'-iodomethyl-2-chloro-biphenyl, 4'-chloromethyl-2-iodo-biphenyl, 4'-chloromethyl-2-bromo-biphenyl, or 4'-chloromethyl-2-chloro-biphenyl.

9. A use of in line analytics for monitoring the reactions as represented on Scheme 1 and/or for adjustment of reaction times or reaction temperatures in the said reactions.

10. The use of in line analytics in accordance with Claim 9 wherein the reaction is a Grignard reaction, in line analytics is FTIR and one or more of the following peaks: 783 cm-1 and 1227 cm-1, 1035 cm-1 , 880 cm-1 , 695 cm-1 is monitored.
